# EUROPEAN PATENT APPLICATION

(11) **EP 3 960 104 A1**
(43) Date of publication of application: **02.03.2022**
(21) Application number: 21165809.1
(22) Date of filing: 30.03.2021
(51) Int. Cl.: A61B 18/12, A61B 18/16, A61B 18/00, A61B 18/14

(54) **BLENDING IRE AND RF ABLATION USING A SINE WAVE GENERATOR**

(30) Priority: 25.08.2020 US 202017001717
(71) Applicant: Biosense Webster (Israel) Ltd, Yokneam, 2066717 (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(57) **Abstract**

A medical apparatus includes a probe configured for insertion into a body of a patient and comprising a plurality of electrodes configured to contact tissue within the body. The medical apparatus further includes an electrical signal generator configured to apply between one or more pairs of the electrodes sinusoidal radio-frequency (RF) signals of first and second types in alternation. The signals of the first type have a first voltage sufficient to cause irreversible electrophoresis (IRE) in the tissue contacted by the electrodes and a first power that is insufficient to thermally ablate the tissue, and the signals of the second type have a second power sufficient to thermally ablate the tissue contacted by the electrodes and a second voltage that is insufficient to cause IRE in the tissue.

## Description

### FIELD OF THE INVENTION

The present invention relates generally to medical equipment, and particularly to apparatus and methods for irreversible electroporation (IRE) and radio frequency ablation (RFA).

### BACKGROUND

Irreversible electroporation (IRE) and radio frequency ablation (RFA) are soft tissue ablation techniques, wherein the ablation is done by inserting a catheter or thin probe into the tissue, with electromagnetic radiation radiating from the tip of the catheter.

In IRE, short bipolar pulses of strong electrical fields are applied to create permanent and hence lethal nanopores in the cell membrane, thus disrupting the cellular homeostasis (internal physical and chemical conditions) . Typical pulse widths are from 0.5 to 5 ps, the pulse frequencies are from 50 kHz to 1 MHz, and pulse amplitudes are from 200 to 2000 V. Cell death following IRE results from apoptosis (programmed cell death) and not necrosis (cell injury, which results in the destruction of a cell through the action of its own enzymes) as in other thermal and radiation-based ablation techniques. IRE is commonly used in tumor ablation in regions where precision and conservation of the extracellular matrix, blood flow and nerves are of importance.

In RFA, a high-power alternating current is applied to tissue so that the heat generated by the current ablates the tissue. RFA is applied, for example, in ablating electrical conduction pathways of the heart, tumors, and other dysfunctional tissues. RFA typically uses currents with amplitude in the range of 0 to 200 V and frequency in the range of 350-500 kHz.

### SUMMARY

Embodiments of the present invention that are described hereinbelow provide improved systems and methods for irreversible electroporation and radio frequency ablation.

There is therefore provided, in accordance with an embodiment of the present invention, a medical apparatus, which includes a probe, which is configured for insertion into a body of a patient and which includes a plurality of electrodes configured to contact tissue within the body. The medical apparatus also includes an electrical signal generator configured to apply between one or more pairs of the electrodes sinusoidal radio-frequency (RF) signals of first and second types in alternation. The signals of the first type have a first voltage sufficient to cause irreversible electrophoresis (IRE) in the tissue contacted by the electrodes and a first power that is insufficient to thermally ablate the tissue, and the signals of the second type have a second power sufficient to thermally ablate the tissue contacted by the electrodes and a second voltage that is insufficient to cause IRE in the tissue.

In a disclosed embodiment, the electrical signal generator is further configured to apply the signals of the first type without the alternation with the signals of the second type.

In a further embodiment, the electrical signal generator is configured to apply the signals of the second type without the alternation with the signals of the first type.

In another embodiment, the electrical signal generator is configured to apply the signals responsively to a temperature measured by a temperature sensor on the probe.

In yet another embodiment, the probe is configured to contact the tissue in a heart of the patient and to apply the signals so as to ablate the tissue in the heart.

In a disclosed embodiment, the duration of the signals of the first type does not exceed four seconds, while the duration of the signals of the second type exceeds four seconds.

In another embodiment, the first voltage exceeds 500 volts, while the second voltage does not exceed 200 volts.

There is also provided, in accordance with an embodiment of the present invention, a method for inserting a probe into a body of a patient and bringing a plurality of electrodes on the probe into contact with tissue within the body, and applying between one or more pairs of the electrodes sinusoidal radio-frequency (RF) signals of first and second types in alternation, the signals of the first type having a first voltage sufficient to cause irreversible electrophoresis (IRE) in the tissue contacted by the electrodes and a first power that is insufficient to thermally ablate the tissue, and the signals of the second type having a second power sufficient to thermally ablate the tissue contacted by the electrodes and a second voltage that is insufficient to cause IRE in the tissue.

There is additionally provided, in accordance with an embodiment of the invention, medical apparatus, which include a probe configured for insertion into a body of a patient and including a plurality of electrodes configured to contact tissue within the body. An external electrode is configured for application to an external surface of the body. An electrical signal generator is configured to apply between one or more of the electrodes on the probe and the external electrode sinusoidal radio-frequency (RF) signals of first and second types in alternation. The signals of the first type have a first voltage sufficient to cause irreversible electrophoresis (IRE) in the tissue within the body that is contacted by the electrodes and a first power that is insufficient to thermally ablate the tissue. The signals of the second type have a second power sufficient to thermally ablate the tissue within the body that is contacted by the electrodes and a second voltage that is insufficient to cause IRE in the tissue.

### BRIEF DESCRIPTION OF THE DRAWINGS

The present invention will be more fully understood from the following detailed description of the embodiments thereof, taken together with the drawings in which:
Fig. 1 is a schematic pictorial illustration of a medical apparatus used in a medical procedure, in accordance with an exemplary embodiment of the invention; and
Fig. 2 is a schematic illustration of a composite ablation signal, in accordance with an exemplary embodiment of the invention.

### DETAILED DESCRIPTION OF EMBODIMENTS

In some medical procedures, a combination of RFA and IRE can be useful in achieving complete and effective ablation of a target volume of tissue. For example, the physician may want to perform an ablation procedure in which RFA is applied during 20% of the procedure time and IRE is applied during the remaining 80% of the procedure time, or alternatively, during which RFA is applied for most of the procedure time and IRE for only a small fraction. This combination of ablation modalities is commonly realized by utilizing two separate generators: an RF generator providing sinusoidal ablation signals for RFA and an IRE generator providing square-pulse signals for IRE. During the ablation, the two generators are alternatingly switched to couple their signals into the ablation catheter. As two dedicated generators are required, this type of solution is costly.

The embodiments of the present invention that are described herein are based on the realization that a sinusoidal signal of sufficient amplitude (voltage) can be used in IRE, in place of the conventional square-pulse signals. The frequency of the sinusoidal IRE signal can be lower than the frequency of a conventional square-wave IRE signal, but higher than the frequency of the RFA signal. The peak amplitude (voltage) of the sinusoidal IRE signal may be higher than that of conventional square-pulse IRE signals and is selected so that the amplitude of the sinusoidal wave is higher than the threshold required for IRE ablation over a period sufficient for completing the IRE ablation.

Consequently, the same sine wave generator, with suitable adjustment, can be used in generating the waveforms for both the RFA and IRE parts of a procedure: For IRE, the frequency and amplitude of the signal are chosen to provide, as previously described, a sufficiently long period at an amplitude that enables IRE; for RFA, the amplitude of the signal is decreased and the frequency adjusted, for example to a typical value of 480 kHz. The delivery parameters of the signals are adjusted, for example by setting respective signal durations and duty cycles, so that the average power of the IRE signals is insufficient to thermally ablate the tissue, in contrast to the RFA signals, which have a lower peak voltage but average power sufficient to thermally ablate the tissue. For example, the IRE signals may be applied over periods between 100 ms and 4 sec, with duty cycles less than 10%, or even about 1%, whereas the RFA signals are applied continuously over periods of 4-90 sec. Thus, the energy delivered to the tissue by the IRE signal is much lower than the energy delivered by the RFA signal, the higher amplitude of the IRE signal notwithstanding. Consequently, the primary effect of the IRE signal is due to high-voltage induced nanopores in the cell membranes, whereas RFA generates heat in the tissue, and thus ablates it.

Thus, the disclosed embodiments address the requirements for combining RFA and IRE by providing a medical apparatus comprising a versatile electrical signal generator operating in conjunction with a probe, such as a catheter with multiple electrodes, which is inserted into the body of a patient so that the electrodes contact tissue within the body. The signal generator provides sinusoidal radio frequency (RF) signals of first and second types in alternation. The signals of the first type have a voltage sufficient to cause IRE in the tissue contacted by the electrodes, while the power of the signals is insufficient to thermally ablate the tissue. The signals of the second type have a power sufficient to thermally ablate the tissue contacted by the electrodes, while their voltage is insufficient to cause IRE in the tissue. In the embodiments that are described further hereinbelow, the signals are applied between one or more pairs of the electrodes on the probe. Alternatively or additionally, the apparatus and methods described herein may be adapted, *mutatis mutandis,* to apply such signals between one or more electrodes on the probe and an external electrode, such as a back patch electrode, which is applied to the body surface.

In the disclosed embodiments, the physician performing an ablation procedure may separately set the amplitudes and the frequencies of the IRE and RFA signals produced by the single signal generator, as well as selecting the temporal mix (durations) of these signals. Thus, the electrical signal generator provides the signals for the two types of ablation by a simple adjustment of the operating parameters of the generator, without the need for two separate signal generators.

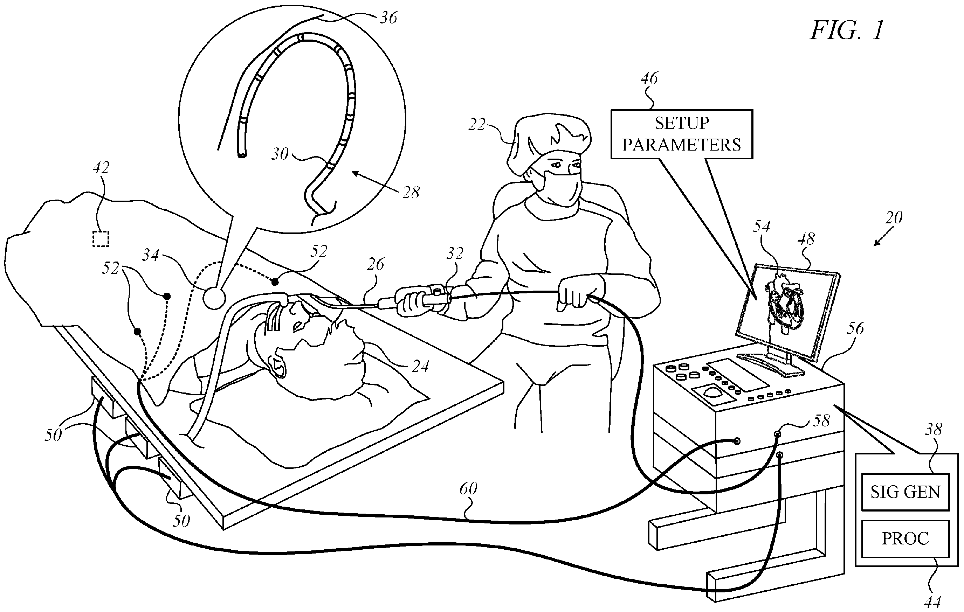

Fig. 1 is a schematic pictorial illustration of a medical apparatus 20 in the course of a medical procedure combining IRE and RFA, in accordance with an embodiment of the invention. A physician 22 performs the procedure on a subject 24, using an ablation catheter 26 whose distal end 28 comprises multiple ablation electrodes 30.

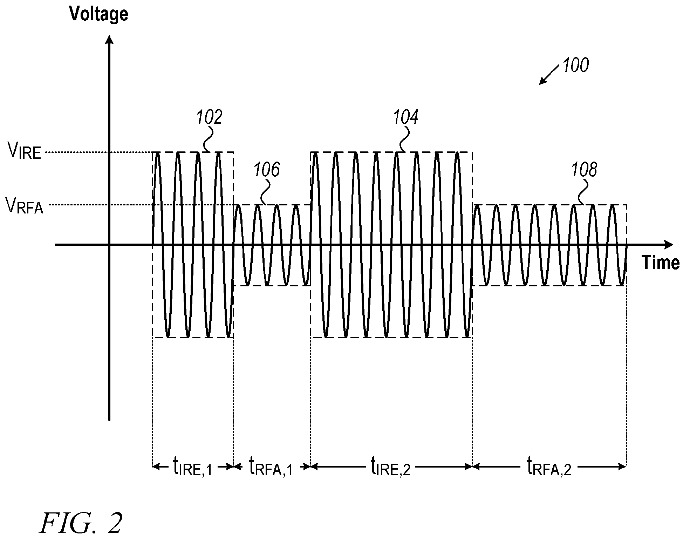

To begin the procedure, physician 22 inserts catheter 26 into subject 24, and then navigates the catheter, using a control handle 32, to an appropriate site within, or external to, a heart 34 of subject 24. The physician brings distal end 28 into contact with tissue 36, such as myocardial or epicardial tissue, of heart 34. Next, an electrical signal generator (SIG GEN) 38 generates multiple sinusoidal signals, comprising alternating IRE signals and RFA signals, as explained below with reference to Fig. 2. Signals are carried through catheter 26, over different respective channels, to ablation electrodes 30, which apply the signals to tissue 36 of subject 24.

In a unipolar RF ablation, the currents of signals flow between ablation electrodes 30 and an external electrode, or "return patch" 42, which is coupled externally between subject 24, typically on the skin of the subject's torso, to SIG GEN 38. In a bipolar RF ablation the currents of the signals flow between pairs of ablation electrodes 30. In an IRE ablation, bipolar signals flow between pairs of ablation electrodes 30. Alternatively, as noted earlier, the RF signals for thermal ablation and IRE may be applied between electrodes 30 and return patch 42.

Medical apparatus 20 further comprises a processor (PROC) 44. Processor 44 receives from physician 22 (or another operator), prior to and/or during the ablation procedure, setup parameters 46 for the procedure. For example, using one or more suitable input devices, such as a keyboard, mouse, or touch screen, physician 22 defines the respective amplitudes and frequencies of a composite signal, comprising IRE and RFA signals in alternation, to be applied to selected electrodes 30, as well as the temporal mix of the IRE and RFA signals, as further explained below with reference to Fig. 2.

Physician 22 may also input, using the above mentioned input devices, additional setup parameters 46, such as a maximum power, a maximum current amplitude, a maximum voltage amplitude, a duration of the signal, and/or any other relevant parameters. The parameters may be set separately for each ablation signal and/or collectively for all of the signals. In response to receiving setup parameters 46, processor 44 communicates with signal generator 38, so that the signal generator generates the signals in accordance with the setup parameters. Additionally, the processor may display the setup parameters on a display 48 (which may comprise the aforementioned touch screen).

Processor 44 may be further configured to track the respective positions of ablation electrodes 30 during the procedure, using any suitable tracking technique. For example, distal end 28 may comprise one or more electromagnetic position sensors (not shown), which, in the presence of an external magnetic field generated by one or more magnetic-field generators 50, output signals that vary with the positions of the sensors. Based on these signals, the processor may ascertain the positions of the electrodes. Alternatively, for each electrode, processor 44 may ascertain the respective impedances between the electrode and multiple external electrodes 52 coupled to subject 24 at various different locations, and then compute the ratios between these impedances, these ratios being indicative of the electrode's location. As yet another alternative, the processor may use both electromagnetic tracking and impedance-based tracking, as described, for example, in US Patent 8,456,182, whose disclosure is incorporated herein by reference.

In some embodiments, processor 44 ascertains which of ablation electrodes 30 are in contact with the subject's tissue, and causes those electrodes, but not the other electrodes, to deliver signals to the tissue. In other words, the processor may select a subset of channels leading to those electrodes that are in contact with the tissue, and then cause signal generator 38 to apply the signals over the selected subset of channels, but not over the other channels.

In some embodiments, processor 44 displays, on display 48, a relevant image 54 of the subject's anatomy, annotated, for example, to show the current position and orientation of distal end 28. Alternatively or additionally, based on signals received from relevant sensors disposed at distal end 28, the processor may track the temperature and/or impedance of tissue 36, and control signal generator 38 responsively thereto. Alternatively or additionally, the processor may perform any other relevant function for controlling, or otherwise facilitating the performance of, the procedure.

Processor 44 and signal generator 38 typically reside within a console 56, and both the processor and the signal generator may each comprise one or several units. Catheter 26 is connected to console 56 via an electrical interface 58, such as a port or socket. Signals are thus carried from signal generator 38 to distal end 28 via interface 58. Similarly, signals for tracking the position of distal end 28 and/or signals for tracking the temperature and/or impedance of the tissue may be received by processor 44 via interface 58. Magnetic-field generators 56 are connected to console 56 via cables 60.

Processor 44 may typically comprise both analog and digital elements. Thus, processor 44 may comprise multiple analog-to-digital converters (ADCs) for receiving analog signals from catheter 26 and from signal generator 38. Processor 44 may further comprise multiple digital-to-analog converters (DACs) for transmitting analog control signals to signal generator 38 and other system components. Alternatively, these control signals may be transmitted in digital form, provided that signal generator 38 is configured to receive digital control signals. Processor 44 typically comprises digital filters for extracting signals at given frequencies from the received signals.

Typically, the functionality of processor 44, as described herein, is implemented at least partly in software. For example, processor 44 may comprise a programmed digital computing device comprising at least a central processing unit (CPU) and random access memory (RAM). Program code, including software programs, and/or data are loaded into the RAM for execution and processing by the CPU. The program code and/or data may be downloaded to the processor in electronic form, over a network, for example. Alternatively or additionally, the program code and/or data may be provided and/or stored on non-transitory tangible media, such as magnetic, optical, or electronic memory. Such program code and/or data, when provided to the processor, produce a machine or special-purpose computer, configured to perform the tasks described herein.

Notwithstanding the particular type of ablation procedure illustrated in Fig. 1, it is noted that the embodiments described herein may be applied to any suitable type of multi-channel ablation procedure that combines the effects of thermal ablation and electroporation.

Fig. 2 is a schematic illustration of a composite ablation signal 100 generated by signal generator 38, in accordance with an embodiment of the invention.

Ablation signal 100 comprises two kinds of sinusoidal signals: IRE ablation signals 102 and 104, having a voltage of V_{IRE}, a frequency of f_{IRE}, and respective durations of t_{IRE,1} and t_{IRE,2}; and RF ablation signals 106 and 108, having a voltage of V_{FRA}, a frequency of f_{RFA}, and respective durations of t_{RFA,1} and t_{RFA,2}. Composite ablation signal 100 may comprise sinusoidal signals with varying voltages, frequencies and durations, as indicated in Table 1, below. Thus, although IRE ablation signals 102 and 104 are shown in Fig. 2 as having the same voltages and the same frequencies, in other embodiments each signal may have a different voltage, as well as a different frequency. Similarly, RF ablation signals 106 and 108 may have different voltages and different frequencies. Furthermore, although the durations T_{IRE} and t_{RFA} in Fig. 2 are shown to be of similar magnitudes, typically t_{IRE} is much shorter than t_{RFA}, as indicated previously and also in Table 1.

**Table 1: Typical values for the parameters of IRE and RFA signals**

| Parameter | Symbol | Typical values |
|---|---|---|
| IRE signal voltage | V_{IRE} | 500-3000 V |
| IRE signal frequency | f_{IRE} | 50 kHz - 1 MHz (typically higher than f_{RFA}) |
| IRE signal duration | t_{IRE} | <4 s (typically 250 ms) |
| Total IRE energy | E_{IRE} | <100 J |
| RF signal voltage | V_{RFA} | 10 - 200 V |
| RF signal frequency | f_{RFA} | 350 - 500 kHz |
| RF signal duration | t_{RFA} | 4 - 90 s |
| Total RFA energy | E_{RFA} | >100 J |

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

### ASPECTS OF THE INVENTION

1. A method for medical treatment, comprising:
   inserting a probe into a body of a patient and bringing a plurality of electrodes on the probe into contact with tissue within the body; and
   applying between one or more pairs of the electrodes sinusoidal radio-frequency (RF) signals of first and second types in alternation, the signals of the first type having a first voltage sufficient to cause irreversible electrophoresis (IRE) in the tissue contacted by the electrodes and a first power that is insufficient to thermally ablate the tissue, and the signals of the second type having a second power sufficient to thermally ablate the tissue contacted by the electrodes and a second voltage that is insufficient to cause IRE in the tissue.
2. The method according to aspect 1, and comprising applying the signals of the first type without the alternation with the signals of the second type.
3. The method according to aspect 1, and comprising applying the signals of the second type without the alternation with the signals of the first type.
4. The method according to aspect 1, wherein applying the signals comprises controlling the signals responsively to a temperature measured by a temperature sensor on the probe.
5. The method according to aspect 1, wherein inserting the probe comprises contacting the tissue in a heart of a patient using the probe, and wherein applying the signals comprises ablating the tissue in the heart.
6. The method according to aspect 1, wherein applying the signal of the first type comprises applying the signal for a first duration not exceeding four seconds, while applying the signal of the second type comprises applying the signal for a second duration exceeding four seconds.
7. The method according to aspect 1, wherein the first voltage exceeds 500 volts, while the second voltage does not exceed 200 volts.

## Claims

1. A medical apparatus, comprising:
a probe configured for insertion into a body of a patient and comprising a plurality of electrodes configured to contact tissue within the body; and
an electrical signal generator configured to apply between one or more pairs of the electrodes sinusoidal radio-frequency (RF) signals of first and second types in alternation, the signals of the first type having a first voltage sufficient to cause irreversible electrophoresis (IRE) in the tissue contacted by the electrodes and a first power that is insufficient to thermally ablate the tissue, and the signals of the second type having a second power sufficient to thermally ablate the tissue contacted by the electrodes and a second voltage that is insufficient to cause IRE in the tissue.

2. The apparatus according to claim 1, wherein the electrical signal generator is further configured to apply the signals of the first type without the alternation with the signals of the second type.

3. The apparatus according to claim 1, wherein the electrical signal generator is further configured to apply the signals of the second type without the alternation with the signals of the first type.

4. The apparatus according to claim 1, wherein the electrical signal generator is configured to apply the signals responsively to a temperature measured by a temperature sensor on the probe.

5. The apparatus according to claim 1, wherein the probe is configured to contact the tissue in a heart of the patient and to apply the signals so as to ablate the tissue in the heart.

6. The apparatus according to claim 1, wherein the signals of the first type have a first duration that does not exceed four seconds, while the signals of the second type have a second duration that exceeds four seconds.

7. The apparatus according to claim 1, wherein the first voltage exceeds 500 volts, while the second voltage does not exceed 200 volts.

8. A medical apparatus, comprising:
a probe configured for insertion into a body of a patient and comprising a plurality of electrodes configured to contact tissue within the body;
an external electrode configured for application to an external surface of the body; and
an electrical signal generator configured to apply between one or more of the electrodes on the probe and the external electrode sinusoidal radio-frequency (RF) signals of first and second types in alternation, the signals of the first type having a first voltage sufficient to cause irreversible electrophoresis (IRE) in the tissue within the body that is contacted by the electrodes and a first power that is insufficient to thermally ablate the tissue, and the signals of the second type having a second power sufficient to thermally ablate the tissue within the body that is contacted by the electrodes and a second voltage that is insufficient to cause IRE in the tissue.

9. The apparatus according to claim 8, wherein the electrical signal generator is further configured to apply the signals of the first type without the alternation with the signals of the second type.

10. The apparatus according to claim 8, wherein the electrical signal generator is further configured to apply the signals of the second type without the alternation with the signals of the first type.

11. The apparatus according to claim 8, wherein the electrical signal generator is configured to apply the signals responsively to a temperature measured by a temperature sensor on the probe.

12. The apparatus according to claim 8, wherein the probe is configured to contact the tissue in a heart of the patient and to apply the signals so as to ablate the tissue in the heart.

13. The apparatus according to claim 8, wherein the signals of the first type have a first duration that does not exceed four seconds, while the signals of the second type have a second duration that exceeds four seconds, and wherein the first voltage exceeds 500 volts, while the second voltage does not exceed 200 volts.
